# EUROPEAN PATENT APPLICATION

(11) **EP 2 549 226 A1**
(43) Date of publication of application: **23.01.2013**
(21) Application number: 12174563.2
(22) Date of filing: 02.07.2012
(51) Int. Cl.: G01B 11/25, G02B 23/24, G06T 7/00, A61B 1/00, A61B 5/107

(54) **Endoscope and endoscope system**

(30) Priority: 20.07.2011 KR 20110072082
(71) Applicant: Samsung Electronics Co., Ltd., Suwon-si, Gyeonggi-do, 443-742 (KR)
(72) Inventor: Kim, Yeon-ho, Yongin-si, 446-712 Gyeonggi-do (KR); Lee, Seung-wan, Yongin-si, 446-712 Gyeonggi-do (KR); Park, Dong-ryeol, Yongin-si, 446-712 Gyeonggi-do (KR); Won, Jong-hwa, Yongin-si, 446-712 Gyeonggi-do (KR)
(74) Representative: Jacobs, Bart

(57) **Abstract**

An endoscope system and an endoscope (200) includes a patterned light providing unit (210) to provide patterned light having a pattern corresponding to a feature point; first and second capturing units (220, 230) to capture an object onto which the patterned light is irradiated; and a light transmitting unit (240) to transmit the patterned light to the object and transmit light reflected by the object to the first and second capturing units.

## Description

### BACKGROUND

### 1. Field

The following description relates to an endoscope for generating an image including depth information of an object to be captured, and an endoscope system.

### 2. Description of the Related Art

An endoscope is a medical tool that is inserted into a human body in order to enable direct observation of an organ or a body cavity to observe a lesion that may not otherwise be observed without an operation or an incision. An endoscope has a narrow, long insertion portion that is inserted into a body cavity to facilitate observation of an organ in the body cavity. A black and white camera is used in an endoscope to capture parts in a body cavity and thus a lesion in each part may be examined in detail through a captured image. However, as image processing technology develops, the simple black and white camera is being replaced by a high resolution color imaging device so that a lesion may be observed in more detail. Also, a chromo endoscope that captures an image after dyeing a surface of a body cavity with a particular pigment corresponding to a type of a lesion to be identified is being used.

Endoscope development is closely connected to providing a more accurate distinction of a lesion. Accordingly, a three-dimensional endoscope is considered as a leading next generation endoscope technology. A conventional endoscope only provides two-dimensional images, and thus it is difficult to accurately detect a lesion therewith. That is, it is difficult to detect a lesion that has a color similar to that of surrounding tissues even if the lesion protrudes to a height different from that of the surrounding tissues. Thus, research is being widely conducted on development of a three-dimensional endoscope that provides not only two-dimensional images, but also depth information regarding a part to be captured.

### SUMMARY

The following description relates to an endoscope that generates an image including depth information by obtaining depth information of an object to be captured, and an endoscope system.

Additional aspects will be set forth in part in the description which follows and, in part, will be apparent from the description, or may be learned by practice of the presented embodiments.

According to an aspect, an endoscope includes a patterned light providing unit to provide patterned light having a pattern corresponding to a feature point; first and second capturing units to capture an object onto which the patterned light is irradiated; and a light transmitting unit to transmit the patterned light to the object and transmit light reflected by the object to the first and second capturing units.

The light transmitting unit may include: a first light transmitting unit to transmit the patterned light to the object; a second light transmitting unit to transmit a part of the light reflected by the object to the first capturing unit; and a third light transmitting unit to transmit the remaining part of the light reflected by the object to the second capturing unit.

The light transmitting unit may be disposed in an insertion portion that may be inserted into a body cavity.

The light transmitting unit may be formed of a waveguide penetrating into the insertion portion.

At least one of the patterned light providing unit, the first capturing unit, and the second capturing unit may be disposed at a posterior end of the insertion portion.

At least one of the patterned light providing unit, the first capturing unit, and the second capturing unit may be disposed at a lateral end of the insertion portion.

The light transmitting unit may include a common light transmitting unit to transmit the patterned light to the object and transmit a part of the light reflected by the object to the first capturing unit or the second capturing unit.

The common light transmitting unit may include at least one bending portion, and wherein a reflection unit to transmit some of incident light and to reflect some of the incident light is disposed in the bending portion.

The reflection unit may include a half mirror.

A shadow may be formed on an area corresponding to the feature point when patterned light is irradiated onto the object.

At least one of the patterned light providing unit, the first capturing unit, and the second capturing unit may be attachable to and detachable from the light transmitting unit.

According to an aspect, an endoscope system includes an endoscope including a patterned light providing unit to provide patterned light having a pattern corresponding to a feature point; first and second capturing units to capture an object onto which the patterned light is irradiated; and a light transmitting unit to transmit the patterned light to the object and transmit light reflected by the object to the first and second capturing units; and a processor to generate an image including depth information of the object by using the feature point.

The endoscope system may further include a light source unit to provide illumination light to illuminate the object.

The endoscope system may further include a switching unit to switch the illumination light to any one of the patterned light providing unit and the light transmitting unit.

The light transmitting unit may include an illumination transmitting unit to transmit the illumination light to a part to be captured.

The patterned light providing unit may generate the patterned light by blocking a part of the illumination light.

The processor may calculate depth information of the object from a relative location relationship of the feature point comprised in each of images captured by the first and second capturing units.

The endoscope system may further include a lookup table storing the relative location relationship of the feature point and the depth information of the object matched with each other, wherein the processor calculates the depth information of the object by reading the depth information according to the relative location relationship of the feature point from the lookup table.

The processor may communicate with the endoscope in a wired or wireless manner.

### BRIEF DESCRIPTION OF THE DRAWINGS

These and/or other aspects will become apparent and more readily appreciated from the following description of the embodiments, taken in conjunction with the accompanying drawings of which:
FIG. 1 is a block diagram illustrating an endoscope system, according to an embodiment;
FIG. 2 is a block diagram illustrating an endoscope of the endoscope system of FIG. 1;
FIG. 3 is a block diagram illustrating a processor of the endoscope system of FIG. 1;
FIG. 4A is a cross-sectional view illustrating an insertion portion of the endoscope of FIG. 2, according to an embodiment, and FIG. 4B is a schematic view illustrating an optical arrangement of the endoscope of FIG. 2, according to an embodiment;
FIG. 5 is a view illustrating the endoscope of FIG. 2 in which a first capturing unit and a second capturing unit are disposed at lateral ends of the insertion portion of FIG. 4A, according to an embodiment;
FIG. 6 is a view illustrating the endoscope of FIG. 2 in which a first light transmitting unit is integrally formed with a second light transmitting unit, according to an embodiment, and FIG. 7 is a view illustrating the endoscope of FIG. 2 in which the first light transmitting unit is integrally formed with a third light transmitting unit, according to an embodiment; and
FIGS. 8 and 9 are views illustrating the endoscope of FIG. 2 including zoom lenses, according to embodiments.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

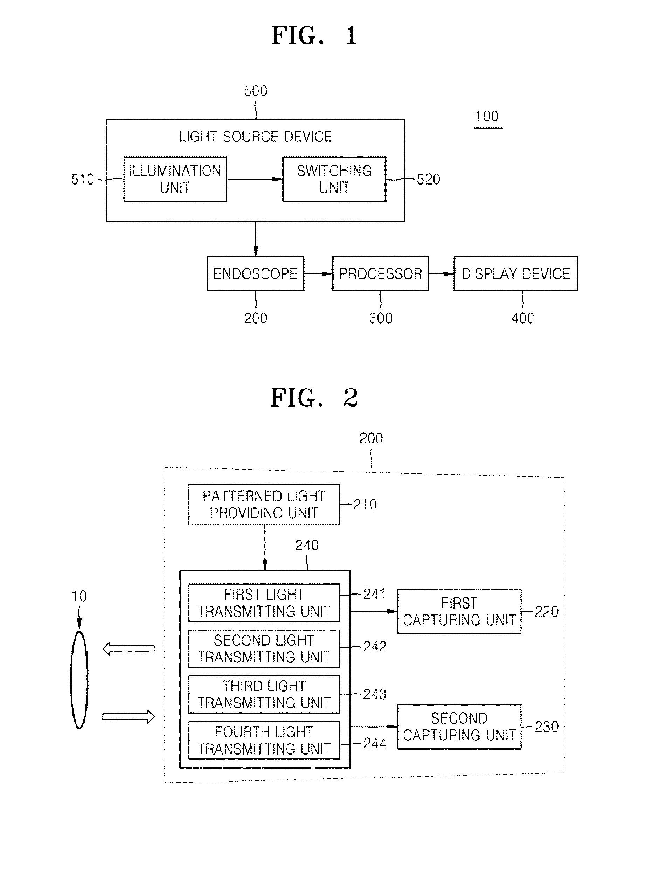

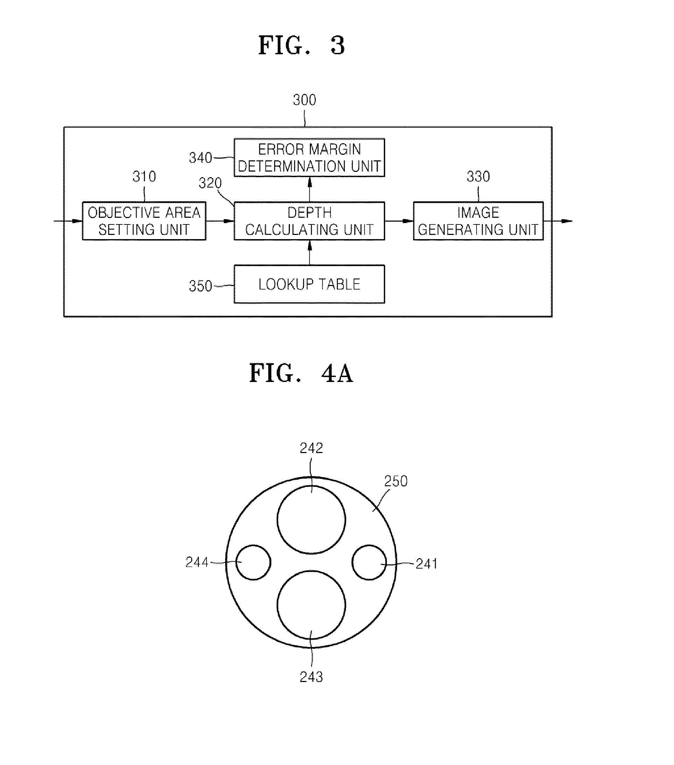

FIG. 1 is a block diagram illustrating an endoscope system 100, according to an embodiment, FIG. 2 is a block diagram illustrating an endoscope 200 of the endoscope system 100 of FIG. 1, and FIG. 3 is a block diagram illustrating a processor 300 of the endoscope system 100 of FIG. 1.

As illustrated in FIG. 1, the endoscope system 100 includes the endoscope 200, the processor 300, a display device 400, and a light source device 500. The endoscope 200 may be connected to the processor 300 in a wired or wireless manner. The endoscope 200 may be connected to the light source device 500 by using an optical fiber, or alternatively, the light source device 500 may be disposed inside the endoscope 200. The processor 300 and the display device 400 may be disposed inside a single housing.

The endoscope 200 is an apparatus that is inserted into a human body in order to capture an object 10, for example, an organ or a body cavity. For example, referring to FIG. 2, the endoscope 200 may include a patterned light providing unit 210 for providing patterned light having any of various patterns corresponding to a feature point of the object 10, first and second capturing units 220 and 230 for capturing the object 10, and a light transmitting unit 240 for transmitting the patterned light to the object 10 and transmitting light reflected by the object 10 to the first and second capturing units 220 and 230. The patterned light providing unit 210, the first capturing unit 220, and the second capturing unit 230 may be formed attachable to and detachable from the light transmitting unit 240.

The patterned light providing unit 210 provides patterned light having a predetermined pattern to the object 10. In this regard, a shadow is formed on a feature point of the object 10 when the patterned light is irradiated onto the object 10. The patterned light providing unit 210 may include an optical filter for blocking a portion of light corresponding to the pattern.

The first and second capturing units 220 and 230 capture the object 10 onto which patterned light is irradiated and may include a complementary metal-oxide semiconductor (CMOS) image sensor or a charge-coupled device (CCD) image sensor. The first and second capturing units 220 and 230 may be disposed spaced apart from each other so as to capture the object 10 from different positions. When the endoscope system 100 generates a three-dimensional image, the first capturing unit 220 may capture an image of the object 10 for a left eye and the second capturing unit 230 may capture an image of the object 10 for a right eye.

The light transmitting unit 240 transmits patterned light to the object 10 and transmits light reflected by the object 10 to the first and second capturing units 220 and 230. For example, the light transmitting unit 240 may include a first light transmitting unit 241 for transmitting the patterned light to the object 10, a second light transmitting unit 242 for transmitting some of the light reflected by the object 10 to the first capturing unit 220, and a third light transmitting unit 243 for transmitting some of the light reflected by the object 10 to the second capturing unit 230. The light transmitting unit 240 may also include a fourth light transmitting unit 244 for transmitting illumination light for illuminating the object 10 to the object 10. The fourth light transmitting unit 244 may be referred to as an illumination transmitting unit.

The first light transmitting unit 241 may be formed to function independently from the second light transmitting unit 242 and the third light transmitting unit 243, or alternatively, the second light transmitting unit 242 or the third light transmitting unit 243 may perform the function of the first light transmitting unit 241. The first through fourth light transmitting units 241 through 244 may be disposed inside an insertion portion 250 (refer to FIG. 4a) of the endoscope 200, wherein the insertion portion 250 has a thin and long shape to be inserted into a body cavity.

The processor 300 receives an image signal of the object 10 from the endoscope 200, analyzes the image signal, and extracts depth information of the object 10 to generate a three-dimensional image. For example, referring to FIG. 3, the processor 300 may include an objective area setting unit 310, a depth calculating unit 320, an image generating unit 330, an error margin determination unit 340, and a lookup table 350.

The objective area setting unit 310 may set an objective area of the object 10 where a depth is to be measured. The objective area may be the entire object 10 or a partial area of the object 10. The objective area may be directly set by a user, or an arbitrary area of the object 10 may be automatically set. The processor 300 does not necessarily include the objective area setting unit 310, and the objective area setting unit 310 is used in consideration of operation efficiency of calculating a depth or to calculate an averaging depth of a predetermined extent of an objective area.

The depth calculating unit 320 may calculate a depth of an objective area. For example, the depth calculating unit 320 may calculate respective depths of shadows formed in objective areas of images captured by the first and second capturing units 220 and 230 by using relative coordinate information regarding the respective shadows and then take an average of the respective depths of the shadows to calculate the depth of the objective area. Alternatively, the depth calculating unit 320 may calculate an average value of the relative coordinate information regarding the respective shadows formed in the objective areas of the images captured by the first and second capturing units 220 and 230 and then calculate a depth corresponding to the average value. When the depth is calculated from the respective coordinate information of the shadows, the depth calculating unit 320 may use the lookup table 350 including a distance value corresponding to the respective coordinate information of the shadows in order to reduce a calculating time and unnecessary operations.

The image generating unit 330 may generate an image of the object 10 by using depth information that is output from the depth calculating unit 320. For example, the image generating unit 330 may generate a stereoscopic image of the object 10 and may display a depth value of a specific area of the object 10 on a corresponding area of the image of the object 10. The generating of the stereoscopic image of the object 10 by using the first and second capturing units 220 and 230 is a general technology in a technological field related to three-dimensional image processing, and thus a detailed description thereof will be omitted here.

The error margin determination unit 340 may determine an error margin that may be generated in depth information provided by the endoscope 200. The error margin determination unit 340 may determine the error margin by using an average depth of the object 10 calculated by the depth calculating unit 320, and a resolution of an image signal that is output from the first and second capturing units 220 and 230. For example, as the average depth of the object 10 increases and as a resolution of an image of the object 10 decreases, the error margin of the depth information increases.

Also, the endoscope system 100 may further include the display device 400 for displaying an image generated by the processor 300 and the light source device 500 for providing illumination light for illuminating the object 10. The illumination light may also function as a source for generating patterned light. For example, the light source device 500 may include an illumination unit 510 for providing illumination light and a switching unit 520 for switching the illumination light to any one of the patterned light providing unit 210 and the light transmitting unit 240. Accordingly, if the switching unit 520 switches the illumination light to the patterned light providing unit 210, the endoscope 200 may illuminate the object 10 with the patterned light, and if the switching unit 520 switches the illumination light to the light transmitting unit 240, the endoscope 200 may illuminate the object 10 with the illumination light.

Hereinafter, optical arrangements of the endoscope 200 will be described with reference to FIGS. 4A to 8.

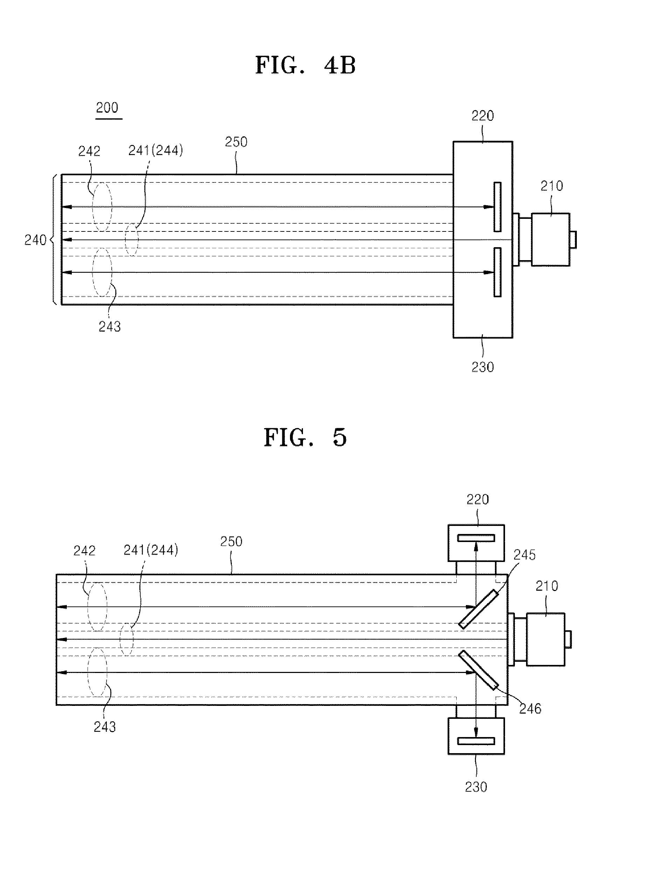

FIG. 4A is a cross-sectional view illustrating the insertion portion 250 of the endoscope 200, according to an embodiment, and FIG. 4B is a schematic view illustrating an optical arrangement of the endoscope 200, according to an embodiment.

As illustrated in FIGS. 4A and 4B, the light transmitting unit 240 may be formed of a waveguide penetrating into the insertion portion 250. For example, the light transmitting unit 240 may be formed of a waveguide penetrating into the insertion portion 250 from an anterior end of the insertion portion 250 toward a posterior end of the insertion portion 250. The light transmitting unit 240 includes the first light transmitting unit 241 for transmitting patterned light to the object 10, the second light transmitting unit 242 for transmitting a part of light reflected by the object 10 to the first capturing unit 220, the third light transmitting unit 243 for transmitting the remaining part of the light reflected by the object 10 to the second capturing unit 230, and the fourth light transmitting unit 244 for transmitting illumination light for illuminating the object 10 to the object 10. The first through fourth transmitting units 241 through 244 may be independently arranged inside the insertion portion 250. The second and third light transmitting units 242 and 243 may respectively include lenses (not shown) for forming an image with the reflected light and guiding the reflected light.

The patterned light providing unit 210, the first and second capturing units 220 and 230, and the light source device 500 (not shown) may be independently disposed at the posterior end of the insertion portion 250. For example, the patterned light providing unit 210, the first capturing unit 220, the second capturing unit 230, and the illumination unit 510 (not shown) may be respectively disposed at posterior ends of the first through fourth light transmitting units 214 through 244. The illumination unit 510 and the patterned light providing unit 210 may be connected to each other by using a switching unit (not shown).

Thus, the endoscope 200 may capture a depth of the object 10 by selectively irradiating patterned light or illumination light onto the object 10. The illumination unit 510 may be directly attached to the insertion portion 250 or may be connected to the insertion portion 250 by using an optical fiber.

Meanwhile, at least one of the patterned light providing unit 210, the first capturing unit 220, and the second capturing unit 230 may be disposed at lateral ends of the insertion portion 250.

FIG. 5 is a view illustrating the endoscope 200 in which the first capturing unit 220 and the second capturing unit 230 are disposed at the lateral ends of the insertion portion 250, according to an embodiment. As illustrated in FIG. 5, the second light transmitting unit 242 and the third light transmitting unit 243 may be formed of a waveguide penetrating into the insertion portion 250 from the anterior end of the insertion portion 250 toward the posterior end of the insertion portion 250. The first and second capturing units 220 and 230 may be respectively disposed at the lateral ends of the insertion portion 250, that is, at the posterior ends of the second light transmitting unit 242 and the third light transmitting unit 243. When the first and second capturing units 220 and 230 are disposed at the lateral ends of the insertion portion 250, the second light transmitting unit 242 and the third light transmitting unit 243 may respectively include bending portions. Incident light may be reflected by a first reflection unit 245 and a second reflection unit 246 that are respectively disposed in the bending portions. The first reflection unit 245 and the second reflection unit 246 may each be configured as a mirror.

FIG. 5 illustrates an optical arrangement of the endoscope 200 in which the first and second capturing units 220 and 230 are disposed at the lateral ends of the insertion portion 250, but the embodiment is not limited thereto. At least one of the patterned light providing unit 210 and the illumination unit 510 may be disposed at the lateral ends of the insertion portion 250. When at least one of the patterned light providing unit 210 and the illumination unit 510 is disposed at the lateral ends of the insertion portion 250, the first light transmitting unit 241 and the fourth light transmitting unit 244 may have a bent waveguide shape in correspondence thereto.

Alternatively, the first light transmitting unit 241 may not be separately disposed in the light transmitting unit 240. Instead, patterned light may be provided to the object 10 through the second light transmitting unit 242 or the third light transmitting unit 243.

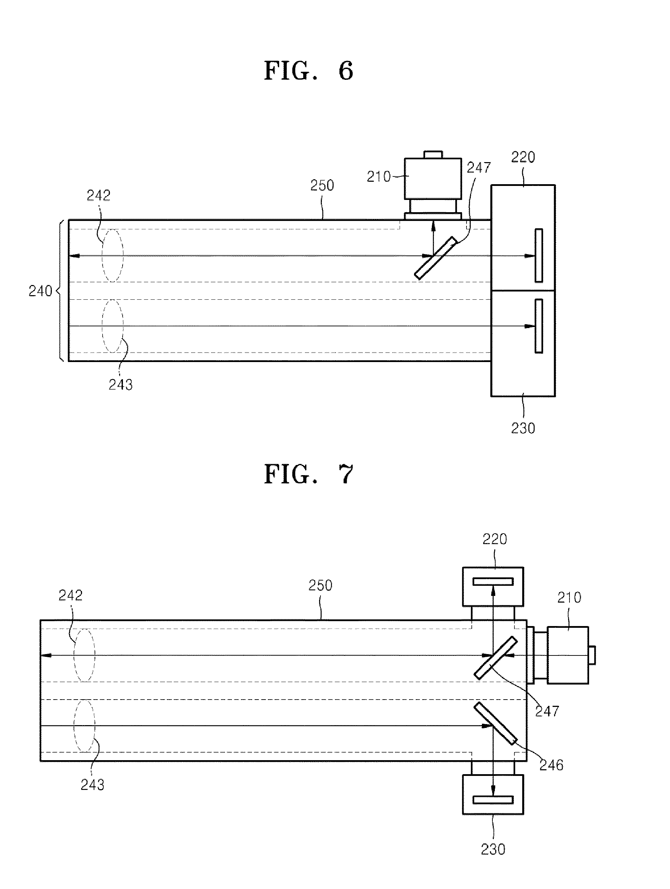

FIG. 6 is a view illustrating the endoscope 200 in which the first light transmitting unit 241 is integrally formed with the second light transmitting unit 242, according to an embodiment. FIG. 7 is a view illustrating the endoscope 200 in which the first light transmitting unit 241 is integrally formed with the third light transmitting unit 243, according to an embodiment.

As illustrated in FIG. 6, the second light transmitting unit 242 may be formed of a waveguide penetrating into the insertion portion 250 from the anterior end of the insertion portion 250 toward one lateral end of the insertion portion 250 and the posterior end of the insertion portion 250. Thus, the second light transmitting unit 242 may include a first posterior end disposed at the one lateral end of the insertion portion 250 and a second posterior end disposed at the posterior end of the insertion portion 250. The patterned light providing unit 210 may be disposed at the one lateral end of the insertion portion 250, that is, at the first posterior end of the second light transmitting unit 242, and the first capturing unit 220 may be disposed at the posterior end of the insertion portion 250, that is, at the second posterior end of the second light transmitting unit 242.

A part of light incident from the patterned light providing unit 210 is reflected by a third reflection unit 247 disposed in the bending portion of the second light transmitting unit 242 and is sent to the object 10, and a part of light incident from the object 10 is transmitted to the first capturing unit 220 by the third reflection unit 247. The third reflection unit 247 may be configured as a half mirror, and locations of the first capturing unit 220 and the patterned light providing unit 210 may be interchanged. The second light transmitting unit 242 may be called a common light transmitting unit.

Furthermore, as illustrated in FIG. 7, the second light transmitting unit 242 may be formed of a waveguide penetrating into the insertion portion 250 from the anterior end of the insertion portion 250 toward one lateral end of the insertion portion 250 and the posterior end of the insertion portion 250. Thus, the second light transmitting unit 242 may include a first posterior end disposed at the one lateral end of the insertion portion 250 and a second posterior end disposed at the posterior end of the insertion portion 250. The first capturing unit 220 may be disposed at the one lateral end of the insertion portion 250, that is, at the first posterior end of the second light transmitting unit 242, and the patterned light providing unit 210 may be disposed at the posterior end of the insertion portion 250, that is, at the second posterior end of the second light transmitting unit 242. Also, the third reflection unit 247 may be disposed in the bending portion of the second light transmitting unit 242.

Furthermore, the third light transmitting unit 243 may be formed of a waveguide penetrating into the insertion portion 250 from the anterior end of the insertion portion 250 toward another lateral end of the insertion portion 250. Thus, the second capturing unit 230 may be disposed at the other lateral end of the insertion portion 250, and the second reflection unit 246 may be disposed in the bending portion of the third light transmitting unit 243.

As such, since patterned light is sent to the object 10 via the second light transmitting unit 242 or the third light transmitting unit 243, there is no need to additionally dispose the first light transmitting unit 241 for sending the patterned light, and thus a width of the insertion portion 250 may be minimized.

Also, the endoscope 200 may further include a zoom lens for precise capturing of the object 10.

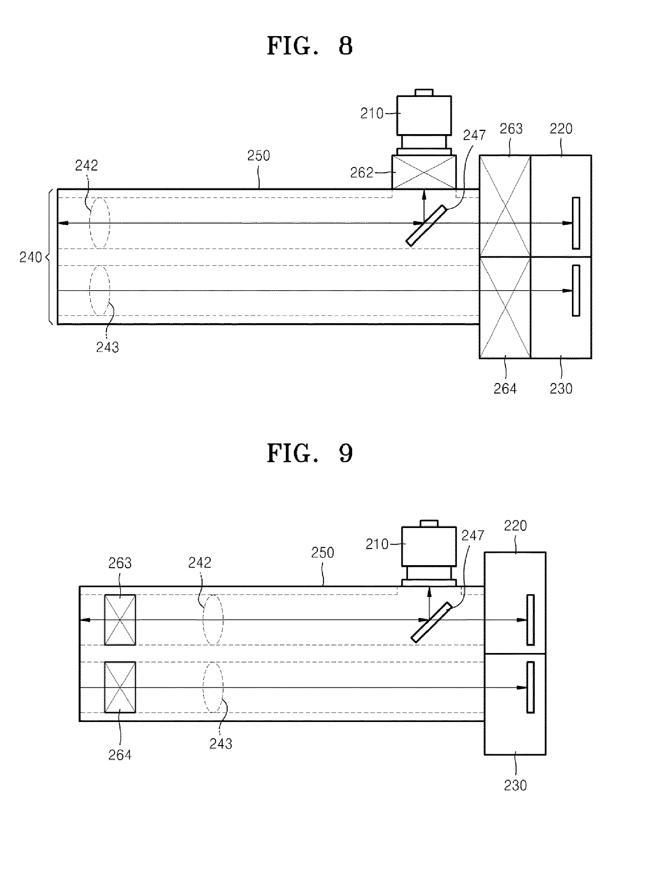

FIGS. 8 and 9 are views illustrating the endoscope 200 including zoom lenses, according to embodiments.

As illustrated in FIG. 8, a first zoom lens unit 262 may be disposed between the patterned light providing unit 210 and the light transmitting unit 240, a second zoom lens unit 263 may be disposed between the first capturing unit 220 and the light transmitting unit 240, and a third zoom lens unit 264 may be disposed between the second capturing unit 230 and the light transmitting unit 240. The first through third zoom lens units 262 through 264 may simultaneously perform zooming in conjunction with one another.

Meanwhile, the patterned light providing unit 210 and the first capturing unit 220 or the second capturing unit 230 may share a zoom lens unit. As illustrated in FIG. 9, the second zoom lens unit 263 may be disposed between an anterior end of the second light transmitting unit 242 and the third reflection unit 247. The third zoom lens unit 264 may be disposed between an anterior end of the third light transmitting unit 243 and the second capturing unit 230. Thus, the endoscope 200 may further precisely capture the object 10 and obtain depth information of the object 10.

As described above, patterned light corresponding to a feature point of the object 10 is used to obtain depth information of the object 10, and thus a process of determining the feature point of the object 10 may be omitted. Also, because the patterned light is transmitted through an existing light transmitting unit, there is no need to increase a width of the endoscope 200.

According to the above description, by providing patterned light having a pattern corresponding to a feature point of an object, depth information of the object may be easily obtained.

Also, since patterned light is transmitted to an object through a light transmitting unit of a capturing unit, a width with respect to an insertion portion of an endoscope can be minimized.

It should be understood that the exemplary embodiments described therein should be considered in a descriptive sense only and not for purposes of limitation. Descriptions of features or aspects within each embodiment should typically be considered as available for other similar features or aspects in other embodiments.

## Claims

1. An endoscope comprising:
a patterned light providing unit to provide patterned light having a pattern corresponding to a feature point;
first and second capturing units to capture an object onto which the patterned light is irradiated; and
a light transmitting unit to transmit the patterned light to the object and transmitting light reflected by the object to the first and second capturing units.

2. The endoscope of claim 1, wherein the light transmitting unit comprises:
a first light transmitting unit to transmit the patterned light to the object;
a second light transmitting unit to transmit a part of the light reflected by the object to the first capturing unit; and
a third light transmitting unit to transmit the remaining part of the light reflected by the object to the second capturing unit.

3. The endoscope of claim 1 or 2, wherein the light transmitting unit is disposed in an insertion portion that may be inserted into a body cavity.

4. The endoscope of claim 3, wherein the light transmitting unit is formed of a waveguide penetrating into the insertion portion.

5. The endoscope of claim 3 or 4, wherein at least one of the patterned light providing unit, the first capturing unit, and the second capturing unit is disposed at a posterior end of the insertion portion.

6. The endoscope of any of the claims 3-5, wherein at least one of the patterned light providing unit, the first capturing unit, and the second capturing unit is disposed at a lateral end of the insertion portion; wherein the light transmitting unit preferably comprises a common light transmitting unit to transmit the patterned light to the object and transmit a part of the light reflected by the object to the first capturing unit or the second capturing unit; wherein the common light transmitting unit preferably comprises at least one bending portion, and wherein a reflection unit to transmit some of incident light and reflect some of the incident light is disposed in the bending portion; wherein the reflection unit preferably comprises a half mirror.

7. The endoscope of any of the previous claims, wherein a shadow is formed on an area corresponding to the feature point when patterned light is irradiated onto the object.

8. The endoscope of any of the previous claims, wherein at least one of the patterned light providing unit, the first capturing unit, and the second capturing unit is attachable to and detachable from the light transmitting unit.

9. An endoscope system comprising:
an endoscope comprising:
a patterned light providing unit to provide patterned light having a pattern corresponding to a feature point;
first and second capturing units to capture an object onto which the patterned light is irradiated; and
a light transmitting unit to transmit the patterned light to the object and transmit light reflected by the object to the first and second capturing units; and
a processor to generate an image including depth information of the object by using the feature point.

10. The endoscope system of claim 9, further comprising a light source unit to provide illumination light to illuminate the object.

11. The endoscope system of claim 10, further comprising a switching unit to switch the illumination light to any one of the patterned light providing unit and the light transmitting unit; and/or wherein the light transmitting unit comprises an illumination transmitting unit to transmit the illumination light to a part to be captured; and/or wherein the patterned light providing unit generates the patterned light by blocking a part of the illumination light.

12. The endoscope system of any of the claims 9-11, wherein the processor calculates depth information of the object from a relative location relationship of the feature point comprised in each of images captured by the first and second capturing units.

13. The endoscope system of claim 12, further comprising a lookup table storing the relative location relationship of the feature point and the depth information of the object matched with each other,
wherein the processor calculates the depth information of the object by reading the depth information according to the relative location relationship of the feature point from the lookup table.

14. The endoscope system of any of the claims 9-13, wherein the processor communicates with the endoscope in a wired or wireless manner.

15. A method for viewing an object with an endoscope, the method comprising:
transmitting patterned light through the endoscope;
irradiating the transmitted light onto the object;
transmitting light reflected from the object back through the endoscope using a first and second transmission medium;
capturing the reflected light from the first transmission medium as a first image;
capturing the reflected light from the second transmission medium as a second image; and
generating a three-dimensional image based on the first and second images.
